Europäisches Patentamt

European Patent Office

Office européen des brevets

(19)

(11) Numéro de publication: **0 099 453**
A2

## (12) DEMANDE DE BREVET EUROPEEN

(21) Numéro de dépôt: 83104623.0

(22) Date de dépôt: 30.01.81

(51) Int. Cl.³: **B 07 C 5/12**
G 07 F 7/06

(30) Priorité: 12.02.80 FR 8003073
06.08.80 FR 8017400

(43) Date de publication de la demande:
01.02.84 Bulletin 84/5

(84) Etats contractants désignés:
AT BE CH DE GB IT LI LU NL SE

(60) Numéro de publication de la demande initiale
en application de l'article 76 CBE: **0 034 088**

(71) Demandeur: SUPERMARKET SYSTEMS
2, chemin du Charme et du Carrosse
F-78470 Saint-Lambert-des-Bois(FR)

(72) Inventeur: Gillet, Guy
54, rue des Glycines Brassioux
F-36130 Deols(FR)

(74) Mandataire: Plaçais, Jean-Yves et al,
Cabinet Netter 40, rue Vignon
F-75009 Paris(FR)

(54) Machine pour la déconsignation d'objets tels que des bouteilles.

(57) Une machine de déconsignation de bouteilles comprend un disque horizontal (101) entraîné en rotation autour d'un axe vertical et dont une face définit une surface de transport sur laquelle on peut déposer des bouteilles (130) à l'entrée (104) de la machine. Ces bouteilles sont transférées vers un dispositif d'identification (112, 120), puis vers un déflecteur (135) qui éjecte les bouteilles reconnues vers l'extérieur du disque d'entraînement (101). Les bouteilles non reconnues poursuivent leur course sur ce disque jusqu'à ce qu'elles parviennent dans une zone (140) au voisinage de l'entrée (104) de la machine, où elles peuvent être reprises par le client. Si elles ne le sont pas, un déflecteur (142) les fait dévier vers une ouverture d'évacuation (146) dans la partie centrale de la machine. Une imprimante (74) produit un ticket indiquant le montant de la somme à rembourser pour les bouteilles reconnues par la machine.

Fig.7

1

## Machine pour la déconsignation d'objets tels que des bouteilles.

L'invention est relative à la déconsignation d'objets tels que des bouteilles à partir d'une reconnaissance de leur forme.

Dans le commerce de détail des liquides, notamment des boissons, les bouteilles en verre constituent, soit des emballages perdus vendus avec le liquide qu'ils contiennent et non réutilisés ultérieurement, soit des emballages consignés qui sont remboursés au client quand celui-ci rapporte les bouteilles au commerçant.

La nécessité s'est fait sentir pour les commerces dits de "grande surface", dans lesquels un grand nombre de bouteilles sont vendues et ramenées, d'automatiser l'identification des bouteilles. C'est pourquoi on a déjà proposé des appareils assurant cette fonction et qui permettent de délivrer un ticket représentant la valeur de la bouteille, ou consigne, et d'effectuer un tri entre les bouteilles consignées, à réutiliser, et les bouteilles non consignées, à détruire.

L'invention a pour objet une machine pour la déconsignation d'objets tels que des bouteilles, du type dans laquelle un convoyeur achemine ces objets entre une ouverture accessible au client et un dispositif d'identification de la forme de ces objets. Elle comprend un dispositif propre à délivrer un ticket imprimé indiquant un montant correspondant au nombre d'objets reconnus, un dispositif permettant de faire le tri entre les objets reconnus et les objets non reconnus, en fonction des résultats de l'identification, et des moyens permettant la reprise par le client des objets non reconnus. Le convoyeur est constitué par une plate-forme horizontale effectuant un trajet bouclé, telle qu'un disque monté rotatif autour de son axe sur lequel peuvent être posés les objets à l'entrée de la machine pour être amenés successivement aux dispositifs d'identification et de tri, les objets non reconnus posés sur le convoyeur poursuivant leur course jusqu'à une zone où ils peuvent être repris par le client et hors de laquelle ils sont évacués s'ils ne sont pas repris. Selon l'invention, les objets non repris sont évacués vers le centre du convoyeur à disque. A cet effet, la machine peut comprendre un déflecteur permettant de dévier, sous l'effet de la rotation du convoyeur, les objets non repris vers une ouverture d'évacuation située dans la partie centrale de ce convoyeur.

La machine est notamment adaptée à la déconsignation de bouteilles, ces dernières étant posées sur le convoyeur à disque avec leur axe vertical.

Lorsque le dispositif d'identification comprend un récepteur sensible à un rayonnement propre à être intercepté par ces bouteilles, le récepteur est placé à un niveau supérieur à la plus grande des bouteilles acceptées par la machine.

La description qui suit est donnée à titre d'exemple en référence aux dessins annexés, dans lesquels :

la figure 1 est une vue schématique en perspective d'une bouteille défilant entre une source et un récepteur;

la figure 2 est une vue de profil d'une bouteille et d'un récepteur, destinée à illustrer les diverses positions relatives de ces éléments ;

la figure 3 est une vue analogue à celle de la figure 2 pour illustrer certains cas particuliers.

la figure 4 est un graphique illustrant les variations d'éclairement du récepteur allongé résultant de la transparence de l'objet à identifier;

la figure 5 représente schématiquement un circuit de commande d'une machine à déconsigner les bouteilles.

la figure 6 est un graphique de courbes types utilisées pour la reconnaissance de la forme de l'objet à identifier;

la figure 7 est un schéma d'une machine à déconsigner les bouteilles mettant en oeuvre les principes de l'invention;

la figure 8 est un schéma plus détaillé d'une partie des circuits de la figure 5;

la figure 9 est un diagramme des signaux mis en oeuvre dans le circuit de la figure 8;

la figure 10 est un schéma d'une page de mémoire.

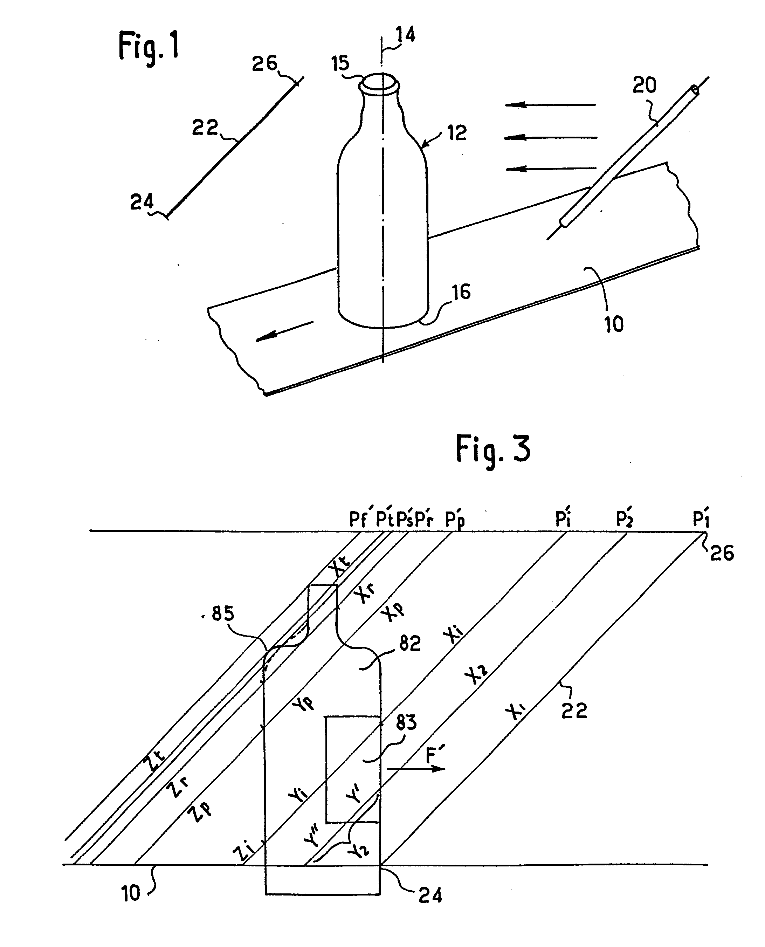

Une machine à déconsigner les bouteilles (figure 1) comprend une plate-forme de convoyeur horizontale 10 propre à défiler dans le sens de la flèche F et à recevoir des bouteilles 12 d'axe vertical 14 dont le fond 16 est posé sur cette plate-forme 10. De part et d'autre de la zone d'espace traversée par la bouteille 12, lorsqu'elle est entraînée par le convoyeur, sont disposés, d'une part, une source de lumière 20 et, d'autre part, un récepteur allongé rectiligne 22 propre à être éclairé par les rayons issus de la source 20 sur toute sa longueur lorsqu'aucun objet tel que la bouteille 12 ne vient intercepter, en partie au moins, ces rayons. La source 20 et le récepteur 22 sont parallèles et inclinés à environ 45° sur la direction de défilement F du convoyeur 10 et dans un plan parallèle à l'axe 14 des bouteilles et à la direction F.

La source 20 est constituée, par exemple, à l'aide d'un tube fluorescent allongé blanc industriel de petit diamètre et d'environ 90 centimètres de long. Il est alimenté à fréquence suffisamment élevée (supérieure à 20 KHz) pour produire un éclairage continu. Une optique, non représentée sur la figure 1, est prévue à l'entrée du récepteur 22 pour former une image des segments de la source 20 non occultés par la bouteille sur la surface sensible du récepteur 22. Le récepteur allongé 22 est composé par exemple d'une barrette de 6,5 millimètres de long comportant 256 photodiodes également espacées entre les extrémités 24 et 26 de ce récepteur, chaque photodiode ayant une longueur de 25 micromètres.

L'agencement relatif de la source 20 et du récepteur 22 est tel qu'une partie du rayonnement issu de la source 20 qui tombe sur le récepteur 22 soit interceptée par une bouteille défilant sur le convoyeur 10 et que, pendant une partie de ce défilement, on puisse définir sur le récepteur 22 au moins trois zones ou segments d'éclairement différent : un segment occulté au moins partiellement par la bouteille 12 qui intercepte une partie du rayonnement tombant sur ce segment, et un premier et un second segments non occultés de part et d'autre du segment occulté, englobant respectivement les extrémités 24 et 26. Des moyens de calage, non représentés, permettent de fixer la distance de la bouteille au récepteur 22 avec une tolérance d'environ 1 centimètre afin que, pour un type de bouteille donné, la transition d'éclairement sur le récepteur correspondant à l'image d'un bord de cette bouteille reste comprise dans un intervalle correspondant à $\pm 1$ photodiode par rapport à une position moyenne donnée, quel que soit l'exemplaire de bouteille du type considéré posé sur le convoyeur.

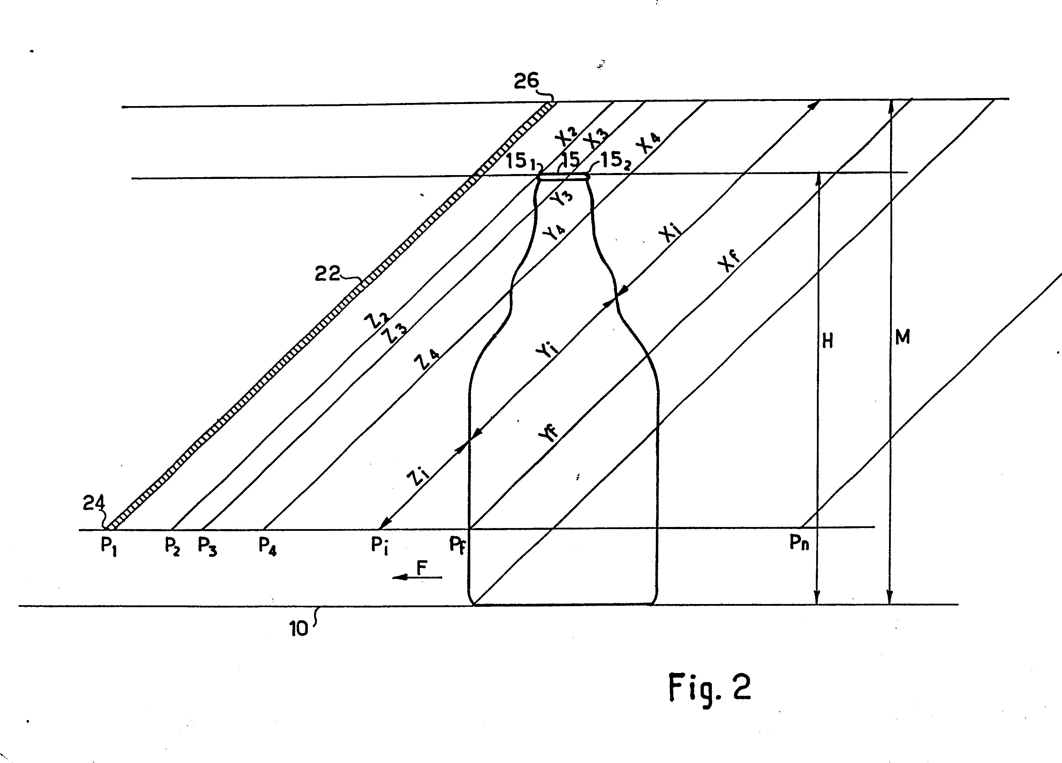

Au cours du défilement de la bouteille 12 sur le convoyeur 10 (figure 2), les positions relatives successives du récepteur 22 représenté par une ligne oblique et de cette bouteille 12 évoluent entre une position $P_1$ dans laquelle la bouteille n'a pas encore pénétré dans le rayonnement atteignant ce récepteur, et une position $P_n$ dans laquelle la bouteille a fini de traverser l'espace entre la source 20 et le récepteur 22. Dans une position intermédiaire $P_i$, la bouteille intercepte une partie du rayonnement issu de la source 20 et définit le segment occulté de longueur Y le long duquel la lumière tombant sur le récepteur est totalement ou partiellement absorbée suivant la transparence de la bouteille. De part et d'autre de ce segment, le premier segment, de longueur X variable à la partie supérieure du récepteur 22, et le deuxième segment, de longueur Z à la partie inférieure du segment du récepteur 22, reçoivent tous deux la totalité du rayonnement qui leur est normalement destiné.

Lors de son défilement, la bouteille balaye (figure 2) une bande d'espace parallèle de largeur égale à sa hauteur H. Le récepteur 22 intercepte cette bande sur toute sa hauteur entre le convoyeur 10 et intercepte notamment la ligne 30 correspondant à la trajectoire du sommet 15 de la bouteille. L'envergure de ce récepteur 22 est telle qu'il intercepte le bord supérieur de toutes les bandes décrites par les bouteilles destinées à être reçues par la machine. L'extrémité supérieure 26 du récepteur 22 est à une distance M de la plate-forme 10 supérieure à la hauteur maximale de ces bouteilles et n'est jamais occultée, même par la plus haute des bouteilles. En revanche, l'extrémité inférieure 24 peut être placée légèrement au-dessus du plan 10, mais suffisamment près pour permettre l'identification des plus petites bouteilles à déconsigner.

Lorsque la bouteille 12 se déplace dans le sens de la flèche F par rapport au récepteur 22, elle pénètre dans le rayonnement atteignant ce récepteur dans la position relative $P_2$. Dans cette position, le bord gauche $15_1$ du sommet 15 de la bouteille, ou bord frontal si l'on considère le sens d'avancement de la flèche F, intercepte le récepteur 22 en un point. On peut relever un segment de longueur $X_2$ et un segment de longueur $Z_2$ dont la somme des longueurs est égale à la longueur du récepteur 22, la longueur $Y_2$ étant nulle.

Lorsque la bouteille progresse et atteint par exemple la position $P_3$, le récepteur 22 intercepte la partie horizontale de l'extrémité 15 de la bouteille, la longueur $X_3$ est égale à $X_2$, tandis que la longueur $Y_3$ n'est plus nulle et $Z_3$ est inférieure à $Z_2$. Lorsque l'extrémité $15_2$ du goulot 15 de la bouteille a franchi le détecteur 22, la valeur du segment X (par exemple $X_4$ pour la position $P_4$ relative de la bouteille et du détecteur) augmente progressivement au fur et à mesure que le mouvement de la bouteille sur le convoyeur 10 se poursuit. La longueur Z s'annule à partir de la position $P_f$ dans laquelle la bouteille rencontre l'extrémité 24 du récepteur 22.

Pour permettre l'identification des bouteilles, les valeurs successives prises par les longueurs des segments X et Z sont relevées et enregistrées en vue de leur exploitation.

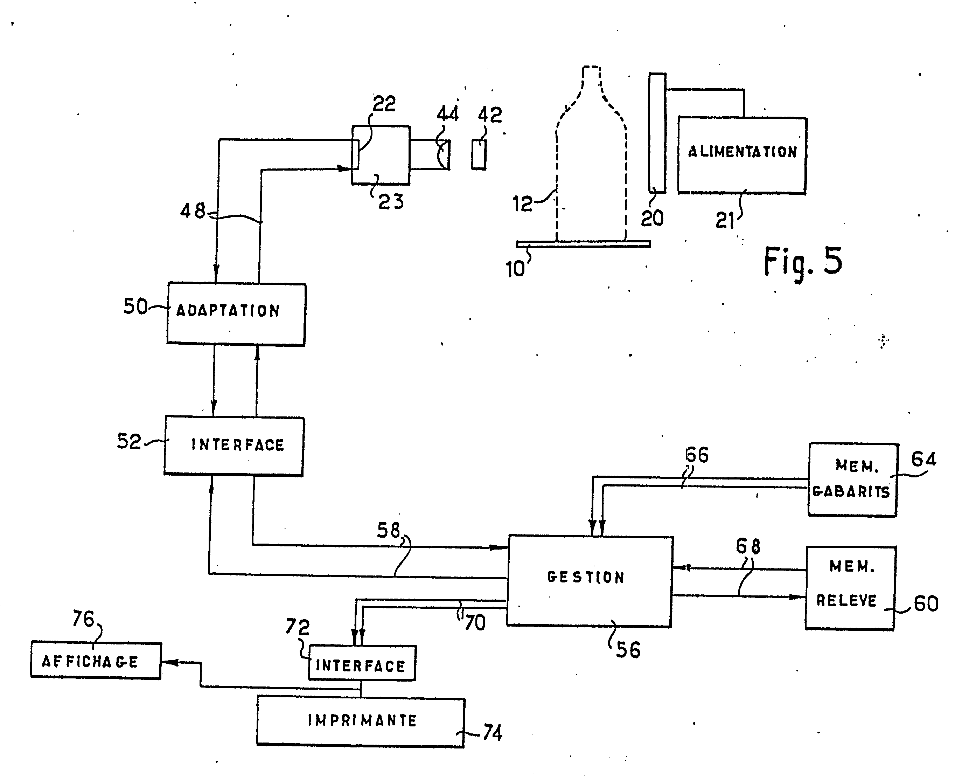

La figure 5 représente schématiquement une machine d'identification équipée d'un tel dispositif de relevé. La bouteille 12 posée sur la plate-forme de convoyeur 10 défile perpendiculairement au plan de la figure devant le tube fluorescent incliné 20 qui est alimenté en tension de fréquence élevée par un bloc d'alimentation 21. La bouteille 12 est à une distance d'environ 10 centimètres de ce tube 20. Elle ne doit pas être trop près de celui-ci pour ne pas engendrer une diffusion trop importante de la lumière au voisinage de ses bords.

A l'opposé du tube 20 par rapport au trajet de la bouteille est monté un boîtier 23 au fond duquel est placée la barrette 22. Une lentille objectif 44 est montée dans une ouverture à l'avant du boîtier 23 pour former une image de la source lumineuse 20 telle qu'occultée par la bouteille 12 sur la barrette de photodiodes 22. Un filtre polarisant 42 placé en avant de l'objectif permet de limiter d'éventuels reflets sur le verre des bouteilles. L'objectif 44, dans cet exemple, a une distance focale de 8,5 millimètres et une ouverture f = 2. Bien entendu, pour la mise en oeuvre de l'invention, il n'est pas nécessaire que la barrette 22 soit dans un plan vertical parallèle à l'axe des bouteilles. En particulier, l'optique qui forme l'image de la source 20 sur la barrette pourrait effectuer un changement d'angle, à l'aide de miroirs par exemple, ou simplement l'axe optique de l'objectif 44 peut être incliné par rapport au plan de la plate-forme 10 pour des raisons qui apparaîtront plus loin. Il est important que la partie utile du récepteur tel que 20 soit inclinée par rapport à la direction de défilement de l'ombre portée de la bouteille sur le récepteur.

La barrette 22 est reliée par une liaison bidirectionnelle 48 à un circuit d'adaptation 50 qui met en forme les impulsions de sortie en série de la barrette 50 correspondant successivement aux niveaux d'éclairement de chacun des éléments photosensibles et transmet un signal binaire en série à un circuit de gestion 56 par une liaison bidirectionnelle 58 à travers une interface 52. Après analyse du signal série par le circuit de gestion 56, les informations de longueur X et Z sont transférées dans

une mémoire de rčlevés 60 par une liaison bidirectionnelle 68. Cette mémoire 60 est une mémoire vive adressable par l'unité de gestion 56.

Le circuit de gestion 56 constitué par un microprocesseur par exemple, est apte à effectuer la comparaison des informations mises en mémoire dans la mémoire 60 et d'informations types correspondant à une collection prédéterminée de bouteilles emmagasinées dans une mémoire de gabarit 64 reliée à cette unité de gestion par une liaison 66. Les résultats de la comparaison des informations dans les mémoires 60 et 64 se traduisent par une décision de rejet ou d'acceptation de chaque bouteille ayant traversé l'espace entre la source 20 et le récepteur 22. Cette décision est transmise par une ligne 70, à travers un circuit d'interface 72, à une imprimante de tickets 74 indiquant, le cas échéant, que la bouteille peut être reprise par le magasin et comportant le prix de consigne contre lequel cette reprise peut être effectuée. Si la bouteille est rejetée, cette information est présentée sur un dispositif d'affichage 76 enjoignant au client de reprendre sa bouteille.

Lors du déplacement d'une bouteille, la procédure d'identification s'effectue en deux étapes successives d'acquisition des mesures des segments X et Z d'une part et d'une analyse des valeurs mesurées pour la reconnaissance proprement dite d'autre part.

La procédure d'acquisition va maintenant être explicitée.

La barrette 22, dans cet exemple, est un réseau intégré de photodiode de type couramment disponible dans le commerce et décrit par exemple dans un article intitulé "Les premiers réseaux intégrés de photodiodes et leurs applications" par J. LESER dans la revue EMI N° 166 du 15 janvier 1973. De tels réseaux sont également fabriqués par la société Reticon Corp. 910 Benicia Ave, Sunnyvale California 94086 USA sous la référence "G-Series Solid State Line Scanners".

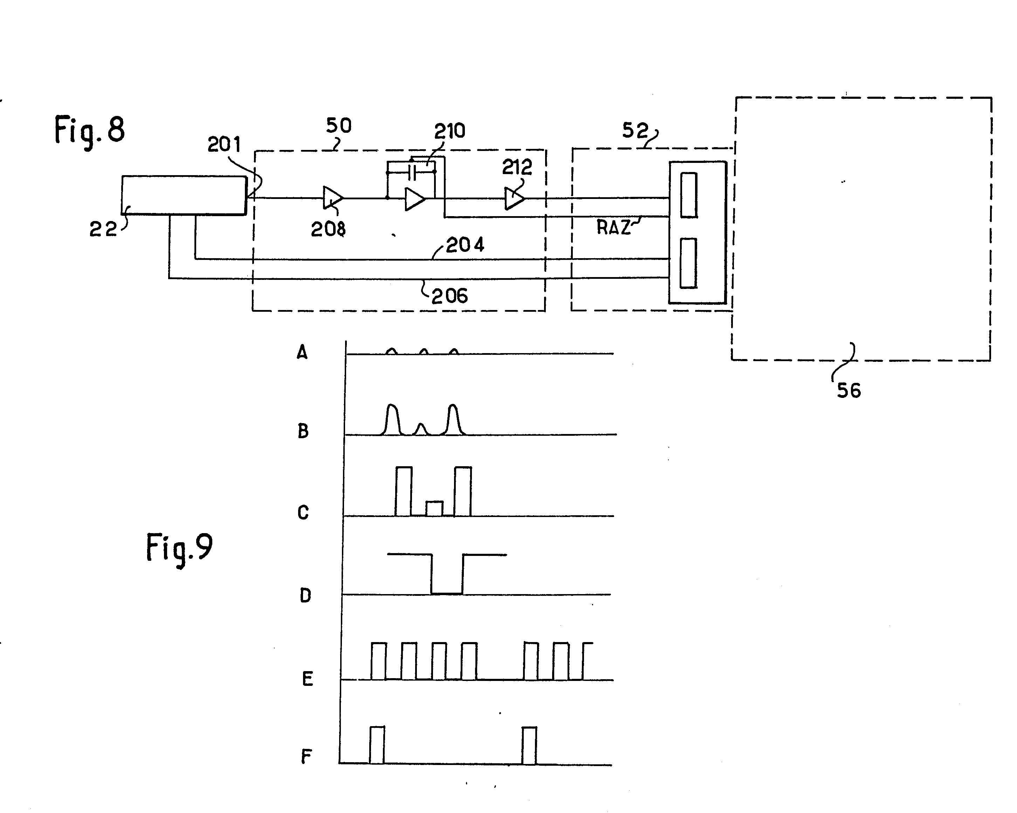

Dans ces dispositifs, chaque photodiode est associée à un condensateur intégré dans le silicium et un registre à décalage également intégré vient recharger séquentiellement ces condensateurs. Plus l'éclairement de la photodiode est fort et plus le condensateur se décharge et plus le courant de, recharge est élevé. Les impulsions de courant de recharge (figure 9A) apparaissent à la sortie 201 de la barrette 22 (figure 8) sous le contrôle d'un signal d'horloge CK (figure 9E) en provenance de l'unité de gestion 56 par une ligne 204 qui commande l'auto-balayage des 256 diodes. Cet auto-balayage est répété 800 fois par seconde sous la commande du signal ST (figure 9F) transmis à partir de l'unité de gestion 56 par une ligne 206 à la barrette 21.

Les impulsions à la sortie 201 de la barrette sont, dans le circuit d'adaptation 50, amplifiées par un amplificateur 208, mises en forme par un intégrateur 210 et transmises à un détecteur de seuil 212 qui délivre un signal à deux niveaux (figure 9D) dont les transitions sont en correspondance des impulsions d'horloge CK (figure 9E) à la mémoire de l'unité de gestion 56 à travers l'interface 52.

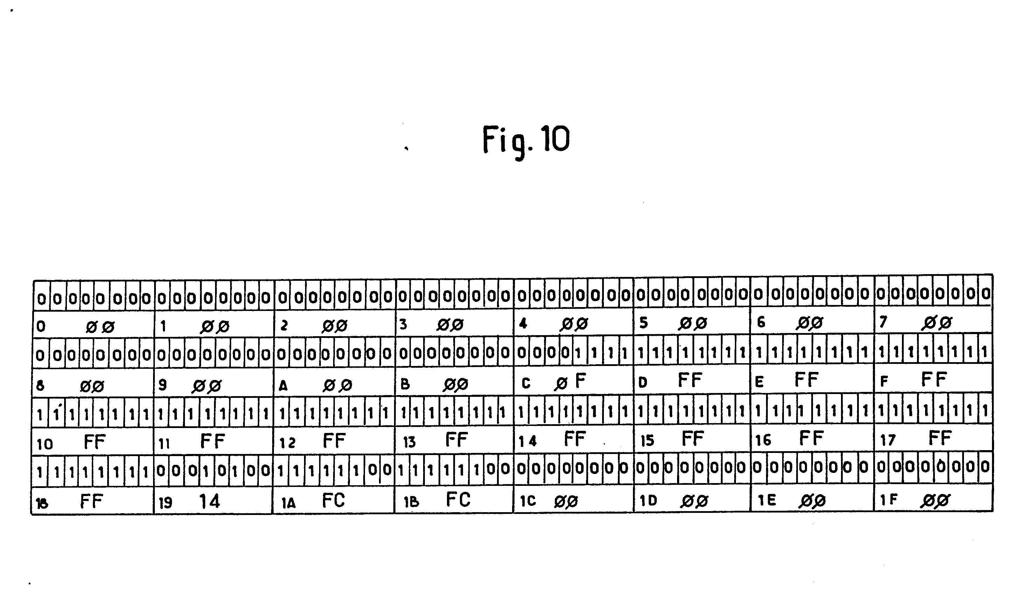

Les signaux ST et CK sont émis pendant le temps où l'unité de gestion est en position d'attente avant qu'une bouteille n'intercepte le rayonnement. Dès qu'au moins une diode est occultée, les signaux issus de chaque balayage (figure 9D) au nombre de 256, sont mémorisés dans 32 octets de mémoire vive, de l'unité de gestion, chaque niveau du signal d'entrée correspondant à un bit 0 ou 1. Un exemple d'une "page" de mémoire contenant ces 32 octets est représenté à la figure 10.

La lecture des valeurs de X et Z est effectuée par l'unité de gestion qui scrute les octets à la suite en partant de l'octet extrême 1F jusqu'à ce qu'il rencontre un octet ayant un contenu non nul, ici 1B. L'unité de gestion scrute ensuite l'octet 1B pour déterminer qu'il possède deux bits nuls, la longueur de X correspondant à $4 \times 8 + 2 = 34$ photodiodes. (Les niveaux zéro dans l'exemple représenté correspondent à des diodes éclairées).

L'unité de gestion procède de la même façon à partir de l'autre extrémité de la page, c'est-à-dire de l'octet 0 pour déterminer que Z : $12 \times 8 + 4 = 100$. Puis, l'unité de gestion scrute les octets entre les octets C et 1B pour déterminer si le contenu de l'un d'eux est différent de FF (8 bits de niveau 1), ce qui correspond à une transparence de la bouteille. Un registre de transparence est incrémenté alors d'un point. En même temps, les valeurs de X et Z ainsi déterminées sont enregistrées dans la mémoire de relevé 60 en plaçant la valeur déterminée pour Z dans une position de cette mémoire dont l'adresse correspond à la valeur déterminée pour X. Si une valeur de Z avait déjà été enregistrée dans cette position, elle est supplantée par la nouvelle valeur.

Ainsi, lorsqu'une bouteille, telle que 12, intercepte les rayons tombant sur le récepteur allongé 22, des couples de valeurs successives X et Z sont mis en mémoire 60, la relation correspondante étant caractéristique de la forme de cette bouteille. On a déterminé en effet que, pour des bouteilles ayant des profils différents, on obtenait des relations différentes entre ces longueurs de segments. Il est donc possible d'identifier une bouteille de forme inconnue en comparant la relation obtenue par le relevé des longueurs correspondant au passage de cette bouteille entre la source et le détecteur et des relations types ou gabarits déjà relevées correspondant aux bouteilles connues.

La relation entre deux des trois longueurs de segments X, Y et Z dépend, pour une forme de bouteille donnée, de l'inclinaison du récepteur 22 par rapport à la direction de défilement de l'ombre portée des bouteilles sur ce récepteur. On a trouvé qu'une inclinaison d'environ 45°, éventuellement légèrement inférieure, donnait des résultats favorables pour des objets ayant la forme de bouteilles. Cette obliquité du récepteur 22 sur la direction de défilement des objets constitue une caractéristique essentielle de la mise en oeuvre de l'invention sans laquelle aucune relation caractéristique de la forme desdits objets ne pourrait être trouvée entre les longueurs de deux des segments du récepteur 22. Cette relation ne dépend pas de la vitesse de défilement de l'objet à identifier, si l'on fait abstraction du temps de fonctionnement éventuel de l'appareillage de lecture et d'analyse (figure 5).

A chaque position de la bouteille devant la source correspond un rapport entre les longueurs des segments X et Z qui dépend seulement de cette position et de la forme de la bouteille. La variation de longueur de l'un ce ces segments Z en fonction de celle de l'autre X, est indépendante du temps et donc de la vitesse. Même si la bouteille venait à reculer puis reprenait son mouvement d'avance, les mesures relevées restent les mêmes pour une bouteille donnée.

Ainsi, pour que l'acquisition des mesures soit effectuée convenablement, il suffit que la bouteille parte d'un point de départ et se rende en un point d'arrivée en passant dans le rayonnement indépendamment de son évolution entre ces deux points.

Les couples de valeurs (figure 2) $X_2$, $Z_2$; $X_3$, $Z_3$; $X_4$, $Z_4$; $X_i$, $Z_i$, etc, dépendent de la forme de la bouteille. Ils dépendent notamment de la hauteur H de cette bouteille et des longueurs $Y_3$, $Y_4$, $Y_i$, du segment variable du récepteur occulté par cette bouteille.

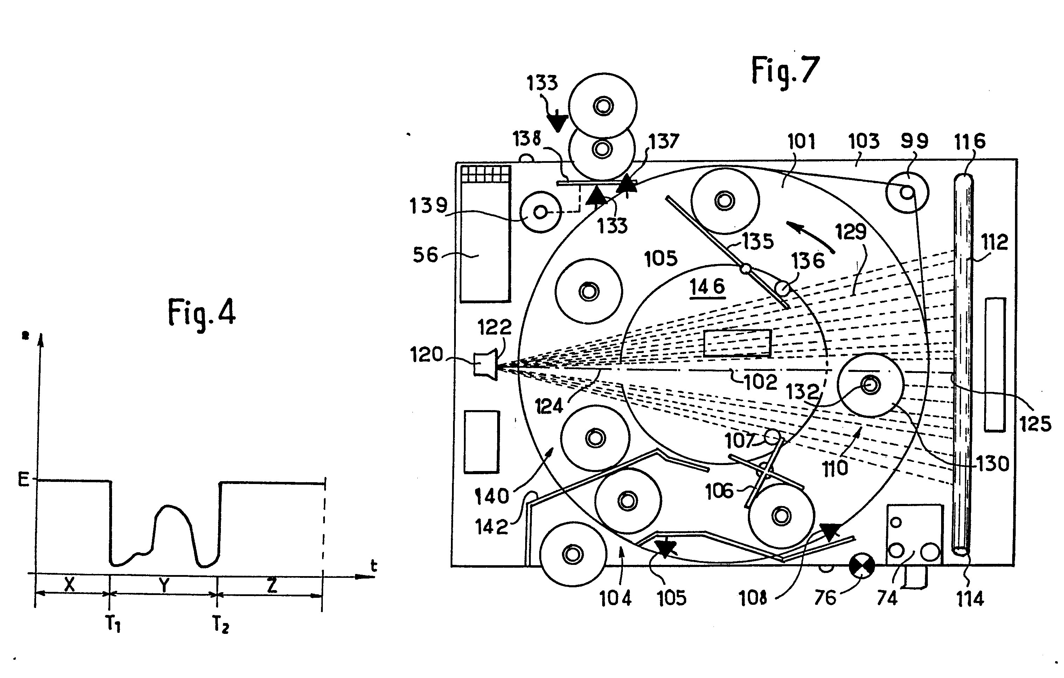

Dans le cas d'objets transparents tels que des bouteilles, on préfère relever les variations conjointes de X et de Z, le degré d'éclairement (ou d'occultation) du segment Y n'étant pas uniforme, et il est souhaitable de pouvoir déterminer dès le moment où la bouteille 12 commence à pénétrer dans le rayonnement entre la source 20 et le récepteur 22, une valeur X et une valeur Z. Sur la figure 3, on a supposé que le mouvement d'une bouteille 82 se produisait en sens inverse, indiqué par la flèche F', de celui de la bouteille 12 de la figure 2. On a représenté les positions relatives $P'_1$, $P'_i$, $P'_p$, .. $P'_f$ du récepteur 22 par rapport à la bouteille 82 au fur et à mesure du défilement, et dans cet ordre. L'inclinaison du récepteur allongé 22 est telle que, lorsque la bouteille défile dans le sens F', elle commence à intercepter le récepteur en un point situé à sa partie inférieure. Lorsque le mouvement de défilement se poursuit, le point d'interception du récepteur par la bouteille se déplace vers le haut le long de ce récepteur, contrairement à ce qui se produisait dans le cas de la figure 2 où le point d'attaque du récepteur 22 par le profil ou l'ombre portée de la bouteille se déplaçait vers le bas, c'est-à-dire vers le convoyeur 10. Quand la bouteille passe à la position relative $P'_2$ dans laquelle le point d'attaque de la bouteille 82 sur le récepteur 22 correspond à la base d'une étiquette 83 de cette bouteille, le segment $X_2$ est bien défini par une ligne du récepteur dont le rayonnement n'est absolument pas intercepté. Au contraire, le degré d'occultation du segment $Y_2$ correspondant est variable. Il se compose, d'une part, d'une partie Y' complètement occultée par l'étiquette 83, et d'une partie Y" recevant des rayons qui ont pu traverser le centre de la bouteille, dans la mesure où celui-ci peut être relativement transparent. Si l'on procédait sans précaution, il ne serait donc pas impossible que l'appareil de détection et d'analyse du récepteur 22 puisse confondre la zone Y" avec un segment du type Z défini précédemment alors même que l'extrémité 24 du récepteur est encore occultée.

Une telle difficulté n'est pas à craindre avec un récepteur dont l'extrémité 20 est positionnée à la distance M de la plate-forme 10 en fonction des critères indiqués précédemment lorsqu'on fait progresser la bouteille à identifier dans le sens de la flèche F, représenté sur la figure 2, puisqu'apparaissent un segment X et un segment Z dès le moment où cette bouteille rencontre le rayonnement destiné au récepteur 22. A partir de cet instant, il est possible de suivre constamment les valeurs de X et Z, sans se préoccuper de la quantité plus ou moins grande de rayonnement qui tombe dans la zone Y, à condition de pouvoir déterminer avec suffisamment de netteté les discontinuités d'éclairement correspondant aux transitions entre cette zone occultée Y et les zones éclairées X et Z.

C'est la raison pour laquelle l'unité de gestion est agencée comme indiqué ci-avant pour commencer cette scrutation des niveaux d'éclairement des points du récepteur 22 par les extrémités respectives de chacun des segments X et Z, c'est-à-dire par les extrémités de ce récepteur 22.

Il est à noter que, même avec des bouteilles transparentes, les bords de celles-ci correspondant aux transitions X, Y et Y, Z sont fort nets. En effet, au bord de la bouteille, l'épaisseur du verre dans la direction des rayons entre la source et le récepteur est très supérieure à cette même épaisseur dans la partie centrale de la bouteille. Il en résulte une absorption de la lumière de la source plus forte par les bords de la bouteille que par la partie centrale de celle-ci. Ce phénomène est illustré par la figure 4 sur laquelle sont figurées, en abscisse, les longueurs mesurées le long du segment récepteur et, en ordonnée, les niveaux d'éclairement $c$ de chacun des points du récepteur. Les points des segments X et Z sont à un niveau uniforme E. La partie centrale du segment Y reçoit également un éclairement qui peut être voisin de E pour un verre blanc assez mince. En revanche, l'éclairement de Y au voisinage des bords $T_1$ et $T_2$ de la bouteille est pratiquement nul. Ainsi, dans la position $P'_i$ (figure 3), les zones $X_i$ et $Z_i$ peuvent être déterminées de façon parfaitement nette.

Pour effectuer le relevé des valeurs X et Z, on préfère déterminer la valeur de Z qui correspond à chaque valeur de X en vue d'obtenir une relation $Z = F(X)$ entre ces deux paramètres comme indiqué précédemment.

En raison notamment de la forme rétrécie de la bouteille 12 vers sa partie supérieure (figure 2), l'inclinaison du segment X du récepteur 22 dont une extrémité s'appuie sur le profil de la bouteille au fur et à mesure de l'avancement de celle-ci est en général plus faible (par rapport à la normale au profil au point d'intersection avec le récepteur) que celle du segment correspondant Z sur la partie du profil correspondante. Il en résulte que la longueur du segment X croît à partir du bord supérieur $15_2$ de la bouteille 12, de façon relativement régulière au fur et à mesure de l'avancement de la bouteille. La valeur de la longueur de X est une fonction sensiblement uniforme du mouvement de cette bouteille.

Au contraire, (figure 3), si l'on observe notamment la position relative $P'_R$ du récepteur 22 par rapport à la bouteille 83, il apparaît qu'avec certains profils de bouteilles tout au moins, le segment Z peut faire l'objet de variations discontinues. Tel est le cas lorsque la bouteille passe par des positions où la ligne du récepteur 22 est tangente au profil de la bouteille 83. Ainsi, par exemple, lorsque la position relative de la bouteille et du récepteur 22 passe de la position repérée par $P'_r$ à la position repérée par $P'_t$, il existe une position $P'_s$ dans laquelle la ligne du récepteur 22 est tangente à l'épaule 85 de cette bouteille, et la valeur de Z subit une discontinuité entre des valeurs $Z_r$ et $Z_t$.

Par ailleurs, on remarque que, même si le profil de la bouteille 82 est tel qu'il n'existe pas de point de tangence tel que 85, l'inclinaison plus forte du segment Z sur le profil correspondant (côté gauche de la bouteille sur les figures 2 et 3) est à l'origine de variations plus rapides de Z en fonction du mouvement d'avance de la bouteille (profil en tirets sur la figure 3). Au contraire, l'extrémité du segment X en appui sur le profil de la bouteille se déplace de façon relativement progressive, d'une manière analogue au doigt d'un palpeur en suivant le mouvement de défilement de la bouteille avec une bonne précision et sans subir de variations brusques ni de rebroussements, comme le montre l'observation des segments $X_p$ à $X_t$ de la figure 3.

C'est cette observation qui est mise à profit pour effectuer le relevé des valeurs de Z en fonction de celles de X en déterminant pour chaque incrémentation de la valeur de X mesurée la valeur de Z correspondante. On obtient donc une suite de valeurs discrètes $Z_i$ en fonction d'une suite continue uniformément croissante de valeurs de $X_i$.

On met en mémoire ces valeurs $Z_i$ dans des positions de la mémoire de relevé 60 dont l'adresse est déterminée directement en fonction du numéro d'ordre de chaque valeur $X_i$. La mémoire 60 comprend 256 positions de mémoire et le relevé du profil d'une bouteille s'effectue, dans le cas du récepteur à réseau de 256 photodiodes, en remplissant régulièrement un sous-ensemble de ces positions en partant de la première pour la valeur $X_2$ lorsque le bord $15_1$ de la bouteille aborde le récepteur 22.

Le circuit de gestion est programmé pour commencer le relevé dès que le circuit de lecture 50 indique qu'une première photodiode entre les extrémités 24 et 26 du récepteur 22 est occultée par le passage d'un objet sur le convoyeur. La mise en mémoire se poursuit jusqu'à ce que le circuit de gestion 56 reçoive de ce circuit de lecture 50 une indication que la longueur $Z_f$ du second segment est égale à zéro (ou à un minimum prédéterminé), cette indication correspondant à la position $P_f$ de la figure 2 dans laquelle la silhouette de la bouteille rencontre l'extrémité inférieure 24 du récepteur 22.

A partir de ce moment, l'acquisition des mesures est terminée et l'unité de gestion 56 procède au traitement d'identification proprement dit. A cet effet, la mémoire de gabarit 64, telle qu'une mémoire REPROM ou à pile C-MOS, contient toute une série d'enregistrements de relations $Z = f(X)$ sous une forme analogue à celle qui vient d'être décrite à propos de la mémoire de relevés 60 et qui correspondent chacune à un type de bouteille déterminé dont la déconsignation est admise. Par des études statistiques préliminaires sur les différentes formes type de bouteilles à déconsigner, on a pu classer certains paramètres (hauteur, diamètre du fût, etc ...) en fonction de leur efficacité pour effectuer le tri entre les gabarits pour identifier une bouteille donnée. L'unité de gestion est agencée pour comparer ces paramètres dans leur ordre de sélectivité décroissante, en vue de minimiser le temps d'identification.

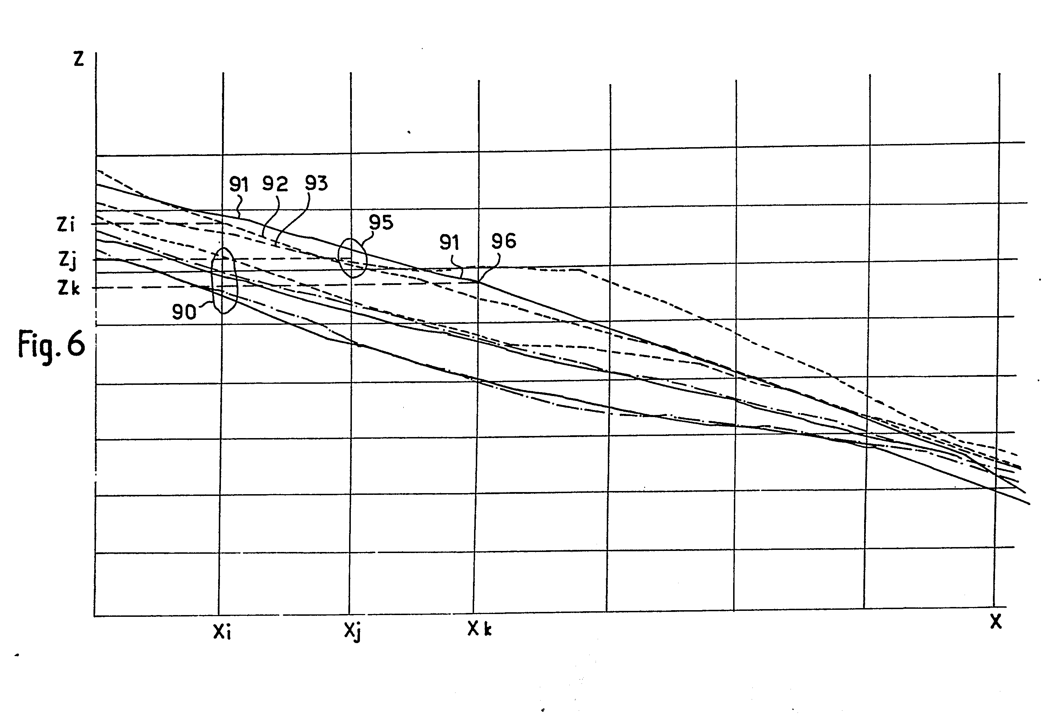

La figure 6 représente une série de courbes correspondant chacune à une relation type ou gabarit caractéristique d'une forme déterminée de bouteille. La détermination de la valeur $X_2$ permet d'effectuer une première comparaison de cette valeur avec toutes les valeurs de départ $X_{2k}$ des relations correspondant à ces différents types de bouteilles. Une première sélection permet donc d'éliminer toutes les courbes dont l'abscisse de départ $X_{2k}$ ne correspond pas, dans les limites de la tolérance de la procédure d'identification, à la valeur $X_2$ enregistrée en mémoire de relevés 60. Un grand nombre de gabarits restants sont ensuite éliminés en déterminant la valeur X qui correspond à $Z = 0$, cette valeur étant indicative du diamètre de la bouteille et en la comparant aux valeurs correspondantes $(Z = 0)$ des gabarits mis en mémoire 64.

Après ces procédures d'élimination préliminaires, on peut sélectionner un certain nombre de points caractérisés par des valeurs $X_i$, $X_j$, $X_k$, etc ., (figure 6) et les valeurs correspondantes $Z_i$, $Z_j$, $Z_k$ mises en mémoire pour déterminer la relation-type à laquelle s'apparente le cas échéant la relation mise en mémoire 60 ou, dans la négative, en l'absence d'une telle relation, pour rejeter la bouteille.

Ainsi, par exemple, le circuit de gestion (56) interroge une position $X_i$ de la mémoire de relevés 60 pour en extraire une valeur $Z_i$ correspondante; puis il interroge successivement les positions $X_i$ correspondant aux différents gabarits dans la mémoire 64 pour effectuer la comparaison des valeurs $Z_{gi}$ correspondantes avec la valeur $Z_i$ extraite de la mémoire 60. Dans le cas de la figure 6, un tel processus de comparaison permet d'éliminer les valeurs $Z_{gi}$ d'abscisse $X_i$ entourées d'un cercle 90 sur la figure, et d'éliminer les relations représentées par les courbes correspondantes sur cette figure.

Seules restent donc en considération trois relations 91, 92 et 93 pour lesquelles les ordonnées $Z_{gi}$ sont relativement proches de l'ordonnée $Z_i$. Le circuit de gestion interroge alors la position de mémoire 60 $X_j$ pour obtenir la valeur $Z_j$, et la mémoire 64 est à nouveau interrogée pour en sortir les ordonnées $Z_{gj}$ des trois gabarits 91, 92 et 93. Dans l'exemple de la figure 6, ces trois valeurs dans le cercle 95 se trouvent être suffisamment proches l'une de l'autre pour ne pas permettre une sélection fiable de l'un de ces gabarits; en conséquence, l'unité de gestion 56 procède à l'interrogation de la position de mémoire $X_k$ dans la mémoire 60 pour déterminer la valeur $Z_k$ et procède ensuite à l'interrogation des positions de mémoire $X_k$ des trois gabarits 91, 92 et 93 de la mémoire 64. Cette dernière interrogation, comme le montre la figure 6, permet de sélectionner sans ambiguité la courbe 91 comme possédant un point 96 d'ordonnée $Z_{gk}$ très voisine de $Z_k$ et d'éliminer en conséquence les deux autres courbes de gabarits 92 et 93. On ne se contente pas de la concordance relevée pour accepter le gabarit 91 comme correspondant à la bouteille à identifier et l'on continue à vérifier la concordance des valeurs Z correspondant à des abscisses X pour le relevé de la mémoire 60 et le gabarit sélectionné jusqu'à épuisement des valeurs mises en mémoire, de façon à rejeter la bouteille comme non consignée si certains des points relevés ne correspondent pas à ce gabarit.

En pratique, on a déterminé qu'un nombre de points relativement limité, par exemple une dizaine, suffisait souvent pour obtenir une bonne sélection ou présélection d'un gabarit parmi différents gabarits des bouteilles à déconsigner.

.Bien entendu, il est possible d'utiliser les mesures effectuées pour améliorer encore la précision de l'identification des objets passés dans la machine, par exemple par des procédures de reconnaissance de formes. On peut également utiliser, dans certains cas, les mesures du niveau d'éclairement correspondant à la partie centrale de la zone occultée Y et enregistrer dans le registre d'éclairement de la mémoire pour effectuer une discrimination supplémentaire entre des bouteilles qui peuvent avoir des formes voisines, mais dont les verres ont des coefficients de transparence très différents (verre teinté et verre blanc, par exemple).

Un mode de réalisation d'une machine conforme à l'invention (figure 7) comprend un disque 101 entraîné en rotation autour de son axe 102 par un moteur 99. Ce disque est monté horizontalement sur un bâti 103 sur un côté duquel est ménagée une ouverture 104 d'admission de bouteilles sur la périphérie du disque·101. Un capteur 105 détecte la présence de la bouteille à l'entrée 104. Un tourniquet 106 bloqué par un électro-aimant 107 arrête la bouteille momentanément tant qu'une autre bouteille est en cours d'identification.

Un capteur 108 détecte la présence d'une bouteille dans le tourniquet 106.

Les bouteilles posées verticalement sur le disque 101 pénètrent ensuite dans une zone 110 de relevé et d'analyse qui comprend notamment une lampe fluorescente allongée 112 qui se projette dans le plan horizontal selon un segment de droite sensiblement tangent à la périphérie du disque 101 et qui est incliné d'environ 45° sur le plan de ce disque. L'extrémité 114 de la lampe 112 située du côté de l'entrée des bouteilles est à un niveau plus élevé que la hauteur maximale des bouteilles défilant sur le disque 101. Dans une direction (vue en plan) diamétralement opposée à la source lumineuse 112 est disposé un détecteur à réseau de photodiodes 120 comportant un objectif 122. Ce détecteur est situé sensiblement au-dessus de la périphérie du disque 101 à une hauteur suffisante pour éviter d'interférer avec la trajectoire de bouteilles posées sur la périphérie de ce dernier. L'axe optique 124 de l'objectif 122 est dirigé vers le milieu 125 de la lampe 112 dans une direction inclinée sur l'horizontale.

La hauteur du milieu 125 de la lampe 112 est inférieure à la hauteur maximale des bouteilles à analyser.

L'objectif 122 forme sur la partie sensible du détecteur 120 une image de la source allongée 112. Lors du passage d'une bouteille dans l'espace 110, l'éclairement de cette image varie en raison de l'interception d'une partie du rayonnement 129 par la bouteille. Lorsque l'axe 132 d'une bouteille telle que 130 sur la figure 7 parvient au voisinage de l'axe optique 124 du détecteur et donc du milieu 125 de la source, l'image de cette dernière sur la partie sensible du détecteur comporte trois segments dont un segment central occulté par la bouteille

**0099453**

130 encadré par deux segments brillants correspondant à chaque extrémité de la source 112. L'unité de gestion 56 contrôle (figure 5) l'analyse de cette image comme il a été indiqué précédemment et les longueurs des segments éclairés sont enregistrées sous la commande du circuit de gestion 56 dans la mémoire de relevé 60.

Après que la bouteille 130 a traversé la zone 110, elle parvient au contact d'un déflecteur mobile 135 commandé par un électro-aimant 136. Si le traitement d'identification indique que la forme de cette bouteille correspond à l'un des profils ou gabarits mis en mémoire, le circuit de gestion provoque, par le blocage de l'électro-aimant 136, la déflexion de la bouteille 130 vers la périphérie du disque de façon à dévier celle-ci en direction d'un poussoir 138 commandé par un moteur 139 en fonction de la détection par un capteur 137 et de capteurs de fin de course avant et arrière 133.

Si la forme de la bouteille 130 n'a pas été identifiée comme correspondant à un des profils types consignés, le déflecteur 135 est libéré par l'électro-aimant 136 et la bouteille poursuit sa rotation à la périphérie du disque 101, passant sous le détecteur 120 pour arriver dans une zone 140 au voisinage de l'ouverture 104 où elle peut être reprise par le client. Si elle ne l'est pas, le disque, en poursuivant sa rotation, la pousse au contact d'un déflecteur 142 qui la repousse radialement vers le centre du disque en direction d'une ouverture 146 au centre de celui-ci par laquelle la bouteille est évacuée.

Le circuit de gestion 56 assure l'enchaînement approprié des opérations de mise en route du disque 101, de la lampe 112, du détecteur 120, la coordination des opérations du tourniquet 106, de l'acquisition des mesures et de la reconnaissance des bouteilles, du déflecteur 135 et du poussoir 138 en fonction des indications des capteurs 105, 108, 133 et 137. Il exploite notamment le résultat de la reconnaissance pour la commande du poussoir 138 et du voyant de refus 76, ainsi que de l'imprimante 74 pour délivrer un ticket imprimé comportant pour chaque client le nombre et le type de bouteilles rendues et le prix correspondant.

Selon une variante, une machine d'identification du type qui vient d'être décrite est appliquée à la constitution d'une trieuse de bouteilles capable de fonctionner à grande vitesse dans des usines d'embouteillage ou des entrepôts de grossistes. La trieuse est équipée d'une pluralité de poussoirs répartis le long du trajet des bouteilles en sortie du rayonnement. Chaque poussoir est monté face à un chemin de convoyage respectif qui débouche sur une table d'accumulation des bouteilles évacuées dans ce chemin par le poussoir respectif. Les poussoirs sont associés à des électro-aimants commandés par le circuit de gestion de façon à réaliser la sélection des bouteilles vers les différents chemins de convoyage en fonction de leurs dimensions et de leurs formes détectées au cours de la phase d'identification.

Selon une variante, à la place de la barrette de photodiodes de la figure 1, on peut utiliser pour le récepteur 22 un photodétecteur surfacique tel que la surface sensible d'une caméra de télévision, (tube VIDICON), associé à des moyens propres à détecter au cours du balayage l'éclairement d'un segment utile de cette surface détectrice le long duquel se forme une image du tube fluorescent présentant un segment occulté par la bouteille. L'analyse de cette image peut s'effectuer par le balayage sous le contrôle de l'unité de gestion en détectant la position dans le temps de signaux correspondant aux points éclairés.

**0099453**

## REVENDICATIONS

1. Machine pour la déconsignation d'objets tels que des bouteilles, comprenant notamment un dispositif d'identification des objets admis dans cette machine et un convoyeur à disque horizontal entraîné en rotation autour de son axe et sur lequel lesdits objets peuvent être déposés par les clients à l'entrée de la machine pour les acheminer vers le dispositif d'identification, puis vers un dispositif de sélection entre les objets reconnus et les objets non reconnus par le dispositif d'identification, ces derniers poursuivant leur course sur ce convoyeur jusqu'en une zone où ils peuvent être repris par le client, caractérisée par un moyen pour l'évacuation desdits objets non repris par le client vers la partie centrale dudit convoyeur à disque.

2. Machine selon la revendication 1, caractérisée en ce que ledit moyen d'évacuation comprend un déflecteur propre à dévier les objets sur le convoyeur à disque entraîné dans ladite zone vers une ouverture au centre du disque.

# Fig.1

# Fig. 3

Fig. 2

Fig.4

Fig.7

0099453

Fig. 5

ALIMENTATION 21

20

12

10

42

44

22

23

48

ADAPTATION 50

INTERFACE 52

58

66

GESTION 56

68

MEM. GABARITS 64

MEM. RELEVE 60

70

INTERFACE 72

IMPRIMANTE 74

AFFICHAGE 76

Fig.6

Fig.8

Fig.9

6 / 7

0099453

# Fig. 10

| | | | | | | | |
|---|---|---|---|---|---|---|---|
| 0 ØØ | 1 ØØ | 2 ØØ | 3 ØØ | 4 ØØ | 5 ØØ | 6 ØØ | 7 ØØ |
| 8 ØØ | 9 ØØ | A ØØ | B ØØ | C ØF | D FF | E FF | F FF |
| 10 FF | 11 FF | 12 FF | 13 FF | 14 FF | 15 FF | 16 FF | 17 FF |
| 18 FF | 19 14 | 1A FC | 1B FC | 1C ØØ | 1D ØØ | 1E ØØ | 1F ØØ |